# EUROPEAN PATENT APPLICATION

(11) **EP 4 714 410 A1**
(43) Date of publication of application: **25.03.2026**
(21) Application number: 24806406.5
(22) Date of filing: 07.05.2024
(51) Int. Cl.: A61F 13/472, A61F 13/475, A61F 13/537, A61F 13/15, C08F 220/18, C08F 220/28, A61L 15/24, A61L 15/20, A61L 15/60

(54) **ABSORPTION PRODUCT AND PREPARATION METHOD THEREFOR**

(30) Priority: 18.05.2023 CN 202310563349
(71) Applicant: The Procter & Gamble Company, Cincinnati, OH 45202 (US)
(72) Inventor: FAN, Jianquan, Guangzhou, Guangdong 510800 (CN); YANG, Jun, Foshan, Guangdong 528500 (CN)
(74) Representative: P&G Patent Germany
(86) International application number: PCT/CN2024/091435
(87) International publication number: WO 2024/235052

(57) **Abstract**

The present invention provides an absorption product, which comprises an absorption core body 1. The absorption core body 1 comprises: an upper permeable layer and a lower permeable layer, and the upper permeable layer and the lower permeable layer have an integrated polymer microporous foam structure, wherein a plurality of first permeable holes are provided in the upper permeable layer, a plurality of second permeable holes are provided in the lower permeable layer, and the aperture of the first permeable holes is larger than that of the second permeable holes. A hydrophilic surface layer 2 is provided on the upper surface of the absorption core body 1, and a water-repellent bottom layer 3 is provided on the lower surface thereof. The absorption core body 1 is obtained by reacting an acrylate monomer, modified acrylate, a cross-linking agent, a photoinitiator, an emulsifier and an initiator, wherein the modified acrylate is obtained by reacting the reaction product of isosorbide and glycidyl ether with acrylic acid. The present invention solves the problems of slow liquid absorption, side leakage and being prone to back seepage due to the accumulation of menstrual blood on the surface of the absorption core body; and has good application prospects.

## Description

### TECHNICAL FIELD

The present invention relates to the technical field of sanitary products, and particularly relates to an absorbent article and a method for preparing the same.

### BACKGROUND

Sanitary napkins are sanitary products invented to help women to have a better quality of life during menstruation, and over many years of development, the design of sanitary napkins has become increasingly diversified, enhancing comfort on various levels.

However, regarding the absorbent core structure used in conventional liquid sanitary napkins, during use, when the menstrual blood on the surface of the absorbent core permeates downward, blood clots tend to accumulate on the surface of the absorbent core, leading to slow absorption by the absorbent core, and resulting in side leakage and easy rewetting of the sanitary napkin.

In summary, there is an urgent need to develop a new technical solution to solve the problems in the prior art and to meet the development demands of the current market.

### SUMMARY

In order to overcome the defects in the prior art, the present invention introduces a large number of active functional groups by adding a modified acrylate having a hyperbranched structure, which can react with an acrylic monomer to obtain an absorbent core with enhanced absorption performance. Also, in the present invention, by adjusting the pore sizes of the upper and lower layers of the absorbent core, the product can generate good capillary action, further improving liquid transfer capacity. The invention solves the problems of slow liquid absorption, side leakage, and easy rewetting caused by the accumulation of menstrual blood on the surface of the absorbent core, and has good application prospects.

An objective of the present invention is to provide an absorbent article, comprising an absorbent core (1), the absorbent core (1) comprising:
an upper permeable layer and a lower permeable layer, wherein the upper permeable layer and the lower permeable layer are each an integrated polymeric microporous foam structure, a plurality of first permeable pores are provided in the upper permeable layer, a plurality of second permeable pores are provided in the lower permeable layer, and the first permeable pores has a pore size greater than that of the second permeable pores;
the absorbent core (1) is provided with a hydrophilic surface layer (2) on an upper surface thereof and a water-repellent bottom layer (3) on a lower surface thereof, and a non-bonded region is provided between the absorbent core (1) and the water-repellent bottom layer (3), thereby forming a liquid storage cavity (6);
adhesive portions are provided on the side of the water-repellent bottom layer (3) away from the absorbent core (1), and are located on two sides of the liquid storage cavity (6);
the edges of the hydrophilic surface layer (2) and the water-repellent bottom layer (3) are connected by a peripheral sealing embossing layer (5), and the hydrophilic surface layer (2) and the water-repellent bottom layer (3) are bonded to each other by an adhesive layer to enclose the absorbent core (1) between the hydrophilic surface layer (2) and the water-repellent bottom layer (3); a vertical pore region is provided on the absorbent core (1), and an adhesive-free region is provided between the water-repellent bottom layer (3) and the vertical pore region, so that the vertical pore region and the water-repellent bottom layer (3) are not bonded to each other, thereby forming the liquid storage cavity (6);
wherein
the absorbent core (1) is obtained by reacting an acrylate monomer, a modified acrylate, a cross-linking agent, a photoinitiator, an emulsifier, and an initiator;
the modified acrylate is obtained by reacting a reaction product of isosorbide and glycidyl ether with acrylic acid.

Further, a method for preparing the modified acrylate comprises the steps of:
S1. adding isosorbide, glycidyl ether, and a catalyst into a solvent, heating the mixture in an inert gas atmosphere to carry out a reaction, cooling the same, and then performing settling, washing, and drying to obtain an intermediate product;
S2. adding the intermediate product and a polymerization inhibitor into a solvent, heating and stirring the mixture, adding a mixed solution of a catalyst and acrylic acid to carry out an reaction, and then performing settling, washing, and drying to obtain the modified acrylate.

Further, in step S1, the temperature of the heating reaction is 60-70°C; in step S2, the heating and stirring temperature is 80-100°C.

Further, the glycidyl ether is selected from one or more of triglycidyl ether and pentaerythritol glycidyl ether.

Further, a method for preparing the absorbent core (1) comprises the steps of:
L1. mixing an acrylate monomer, a modified acrylate, a cross-linking agent, a photoinitiator, and an emulsifier to obtain an oil phase 1, and then adding a water phase A comprising an inorganic salt and a water phase B comprising an initiator to obtain a high internal phase emulsion 1;
L2. mixing an acrylate monomer, a modified acrylate, a cross-linking agent, a photoinitiator, and an emulsifier to obtain an oil phase 2, and then adding a water phase C comprising an inorganic salt and a water phase D comprising an initiator to obtain a high internal phase emulsion 2;
L3. coating the high internal phase emulsion 1 and the high internal phase emulsion 2 onto the upper and lower surfaces of a fabric, respectively, and performing light irradiation, steam treatment, dehydration, and drying to obtain the absorbent core.

Further, the high internal phase emulsion 1 and the high internal phase emulsion 2 are prepared using a continuous emulsification apparatus.

Further, the first permeable pores have a pore size of 60-130 µm, and the second permeable pores have a pore size of 5-40 µm.

Further, a plurality of through-holes (10) penetrating the absorbent core (1) are provided in the vertical pore region, a flow guiding region located on the absorbent core (1) is provided on one side of the vertical pore region, a plurality of arc-shaped flow guiding grooves (11) arranged along the length direction of the absorbent core (1) are provided in the flow guiding region, and the lengths of the plurality of arc-shaped flow guiding grooves (11) present a gradual decrease toward the vertical pore region.

Further, the hydrophilic surface layer (2) and the water-repellent bottom layer (3) are both provided with wing portions (4), the adhesive portions comprises first adhesive strips (12) and second adhesive strips (13) provided on the water-repellent bottom layer (3), and the first adhesive strips (12) are located on the wing portions (4), and the second adhesive strips (13) are symmetrically provided on two sides of the liquid storage cavity (6).

Further, the connection locations between the wing portions (4) and the hydrophilic surface layer (2) and between the wing portions (4) and the water-repellent bottom layer (3) are each provided with an arc-shaped corner (8), and the edge of each arc-shaped corner (8) is provided with an embossed separation region, so that the hydrophilic surface layer (2) and the water-repellent bottom layer (3) are both completely separated from the peripheral sealing embossing layer (5) at the edges of the wing portions (4).

The peripheral sealing embossing layer (5) is provided with at least two layers, each layer of the peripheral sealing embossing layer (5) comprises a plurality of uniformly arranged embossing holes (7), the embossing holes (7) of two adjacent layers of the peripheral sealing embossing layer (5) are aligned along common straight lines, and the number of embossing holes (7) in the at least two layers of the peripheral sealing embossing layer (5) adjacent to the embossed separation region decreases in the direction toward the absorbent core (1), so that an inclined line (9) is formed at an end portion of the at least two layers of the peripheral sealing embossing layer (5) adjacent to the embossed separation region; as a result, the end portion of the peripheral sealing embossing layer at each arc-shaped corner (8) is arranged obliquely, further reducing the development of fractures and preventing tearing and separation of the wing portions.

Further, the inorganic salt is selected from one or more of alkaline earth metal chlorides or sulfates and alkali metal chlorides or sulfates;
the fabric is selected from one of woven fabric and non-woven fabric.

Further, the first acrylate monomer and the second acrylate monomer are alkyl acrylates or alkyl methacrylates, and the alkyl group in the alkyl acrylates or alkyl methacrylates is selected from C4-C20 alkyl groups.

Further, the cross-linking agent is ethylene glycol dimethacrylate.

Further, the light irradiation has a wavelength of 205-450 nm, an intensity of 40-1000 mW/cm², and a duration of 5-180 s.

Further, the initiator is selected from one or more of ammonium persulfate, sodium persulfate, potassium persulfate, and azo initiators.

Further, the photoinitiator is preferably one or more of benzophenone, benzil, thioxanthone, benzyl ketal, α-hydroxyalkylphenone, α-aminoalkylphenone, and acylphosphine oxide.

The emulsifier added in the present invention serves to disperse and emulsify the monomer, providing necessary conditions for the formation and stability of the emulsion. The emulsifier comprises a main emulsifier and an auxiliary emulsifier, and the main emulsifier is a hydrophobic emulsifier, which is beneficial for dispersing polymerized monomers and reducing the solubility thereof in water. The main emulsifier is selected from mono- or poly-stearates, mono- or poly-isostearates, and mono- or poly-oleates of monoglycerol, diglycerol, or polyglycerol having 3-10 glycerol units, further preferably di-glycerol mono-isostearates or oleates and poly-3-glycerol mono-isostearates or oleates. The auxiliary emulsifier is selected from C2-C4 short-carbon-chain and C12-C22 long-carbon-chain aliphatic betaines or quaternary ammonium salts, further preferably propyl dimethyl ammonium bromide and dioctadecyl dimethyl ammonium bromide.

Specifically, the present invention relates to the following embodiments:
1. An absorbent article, comprising:
   a hydrophilic surface layer, a water-repellent bottom layer, and an absorbent core disposed therebetween;
   wherein the absorbent core comprises a high internal phase emulsion foam, the high internal phase emulsion foam comprising an acrylate monomer, a modified acrylate, a cross-linking agent, a photoinitiator, and an emulsifier;
   wherein the modified acrylate is obtained by reacting a reaction product of isosorbide and glycidyl ether with acrylic acid;
   wherein the absorbent core comprises an upper permeable layer and a lower permeable layer, wherein the upper permeable layer comprises a plurality of first permeable pores having a first pore size, and the lower permeable layer comprises a plurality of second permeable pores having a second pore size, wherein the first pore size is greater than the second pore size.
2. The absorbent article of embodiment 1, wherein the glycidyl ether is selected from one or more of triglycidyl ether and pentaerythritol glycidyl ether.
3. The absorbent article of any one of the preceding embodiments, wherein the emulsifier comprises a main emulsifier and an auxiliary emulsifier.
4. The absorbent article of embodiment 3, wherein the main emulsifier is selected from mono- or poly-stearates, mono- or poly-isostearates, and mono- or poly-oleates of monoglycerol, diglycerol, or polyglycerol having 3-10 glycerol units, preferably diglycerol mono-isostearates or oleates and poly-3-glycerol mono-isostearates or oleates.
5. The absorbent article of embodiment 4, wherein the auxiliary emulsifier is selected from C2-C4 short-carbon-chain and C12-C22 long-carbon-chain aliphatic betaines or quaternary ammonium salts, preferably propyl dimethyl ammonium bromide and dioctadecyl dimethyl ammonium bromide.
6. The absorbent article of any one of the preceding embodiments, wherein the first pore size is from 60 µm to 130 µm.
7. The absorbent article of any one of the preceding embodiments, wherein the second pore size is from 5 µm to 40 µm.
8. The absorbent article of any one of the preceding embodiments, wherein a method for preparing the modified acrylate comprises the steps of:
   a. adding isosorbide, glycidyl ether, and a catalyst into a solvent, heating the mixture in an inert gas atmosphere to carry out a reaction, cooling the mixture, and then settling, washing, and drying the cooled mixture to obtain an intermediate product; and
   b. adding the intermediate product and a polymerization inhibitor into a solvent, heating and stirring the mixture, adding a mixed solution of a catalyst and acrylic acid, carrying out an reaction and performing settling, washing, and drying to obtain the modified acrylate.
9. The absorbent article of embodiment 8, wherein in step (a), the temperature of the heating reaction is from 60°C to 70°C, and wherein in step (b), the temperature of heating and stirring is 80-100°C.
10. The absorbent article of any one of the preceding embodiments, wherein the water phase comprises an initiator; wherein the initiator is selected from one or more of ammonium persulfate, sodium persulfate, potassium persulfate, and azo initiators.
11. The absorbent article of any one of the preceding embodiments, wherein the photoinitiator is one or more of benzophenone, benzil, thioxanthone, benzyl ketal, α-hydroxyalkylphenone, α-aminoalkylphenone, and acylphosphine oxide.
12. A method for preparing an absorbent core, comprising the steps of:
   a. mixing an acrylate monomer, a modified acrylate, a cross-linking agent, a photoinitiator, and an emulsifier to form an oil phase;
   b. adding a water phase to the oil phase to form a first high internal phase emulsion;
   c. depositing the first high internal phase emulsion on a surface;
   d. exposing the first high internal phase emulsion to light treatment;
   e. placing the first high internal phase emulsion into a steam tunnel oven; and
   f. dehydrating and drying the high internal phase emulsion to obtain the absorbent core;
   wherein the modified acrylate is obtained by reacting a reaction product of isosorbide and glycidyl ether with acrylic acid.
13. The method of embodiment 12, wherein the glycidyl ether is selected from one or more of triglycidyl ether and pentaerythritol glycidyl ether.
14. The method of embodiment 12 or 13, wherein the water phase comprises an initiator; wherein the initiator is selected from one or more of ammonium persulfate, sodium persulfate, potassium persulfate, and azo initiators.
15. The method of any one of embodiments 12 to 14, wherein the oil phase comprises 60 parts by weight of isooctyl acrylate, 15 parts by weight of the modified acrylate, 20 parts by weight of the cross-linking agent, 1 part by weight of the photoinitiator, and 5 parts by weight of the emulsifier.
16. The method of any one of embodiments 12 to 15, wherein a method for preparing the modified acrylate comprises the steps of:
   a. adding isosorbide, glycidyl ether, and a catalyst into a solvent, heating the mixture in an inert gas atmosphere to carry out a reaction, cooling the mixture, and then settling, washing, and drying the cooled mixture to obtain an intermediate product; and
   b. adding the intermediate product and a polymerization inhibitor into a solvent, heating and stirring the mixture, adding a mixed solution of a catalyst and acrylic acid, carrying out an reaction and performing settling, washing, and drying to obtain the modified acrylate.
17. The method of any one of embodiments 12 to 16, further comprising: mixing a second acrylate monomer, a second modified acrylate, a second cross-linking agent, a second photoinitiator, and a second emulsifier to form a second oil phase; and adding a second water phase to the second oil phase to form a second high internal phase emulsion.
18. The method of embodiment 17, wherein the second high internal phase emulsion is deposited on the first high internal phase emulsion.

The beneficial effects of the present invention are as follows:
1. The present invention uses a modified acrylate to compound with an acrylic monomer and a cross-linking agent to prepare an absorbent core with enhanced adsorption performance. On one hand, the modified acrylate has a hyperbranched structure, so that a more three-dimensional cross-linking structure can be formed during polymerization. On the other hand, the present invention introduces a large number of organic functional groups, including hydroxyl, ester, epoxy, etc., that can generate strong intermolecular interactions and facilitate synergistic effects with ingredients such as emulsifiers, thereby promoting construction of a more stable and uniform structure. As a result, the product has higher porosity and a dense microporous structure, thereby achieving superior three-dimensional fluid storage functionality.
2. The absorbent core in the present invention is composed of an upper permeable layer and a lower permeable layer. A plurality of first permeable pores in the upper permeable layer and a plurality of second permeable pores in the lower permeable layer are matched with each other, and the permeable pores are cross-linked with each other. As the first permeable pores have a larger pore size than that of the second permeable pores, menstrual blood, on the surface of the absorbent core, enters the upper permeable layer having the large-sized first permeable pores and is then quickly dispersed into the lower permeable layer, so that the menstrual blood is quickly and evenly dispersed and stored in the lower permeable layer, ensuring that blood does not accumulate on the surface of the sanitary napkin, and preventing slow liquid absorption caused by menstrual blood buildup on the surface of the absorbent core. Moreover, the liquid storage cavity is provided between the absorbent core and the water-repellent bottom layer, so that in the case of a large amount of menstrual blood, the menstrual blood quickly permeates downward into the liquid storage cavity, and is stored by the liquid storage cavity and then reabsorbed by the absorbent core, thereby effectively preventing menstrual blood rewetting. In addition, the adhesive portions enable the absorbent article to be easily attached to underwear, and since the adhesive portions are located on two sides of the liquid storage cavity, the liquid storage cavity is prevented from being torn and leaking during removal of the absorbent article.

### BRIEF DESCRIPTION OF THE DRAWINGS

FIG. 1 is a schematic front structural view of an absorbent article of the present invention.
FIG. 2 is a schematic rear structural view of the absorbent article of the present invention.
FIG. 3 is a schematic cross-sectional structural view of the absorbent article of the present invention.
FIG. 4 is an enlarged structural schematic diagram of position A in FIG. 1.
   Reference numerals: 1-absorbent core, 2-hydrophilic surface layer, 3-water-repellent bottom layer, 4-wing portion, 5-peripheral sealing embossing layer, 6-liquid storage cavity, 7-embossing hole, 8-arc-shaped corner, 9-inclined line, 10-through-hole, 11-arc-shaped flow guiding groove, 12-first adhesive strip, 13-second adhesive strip.

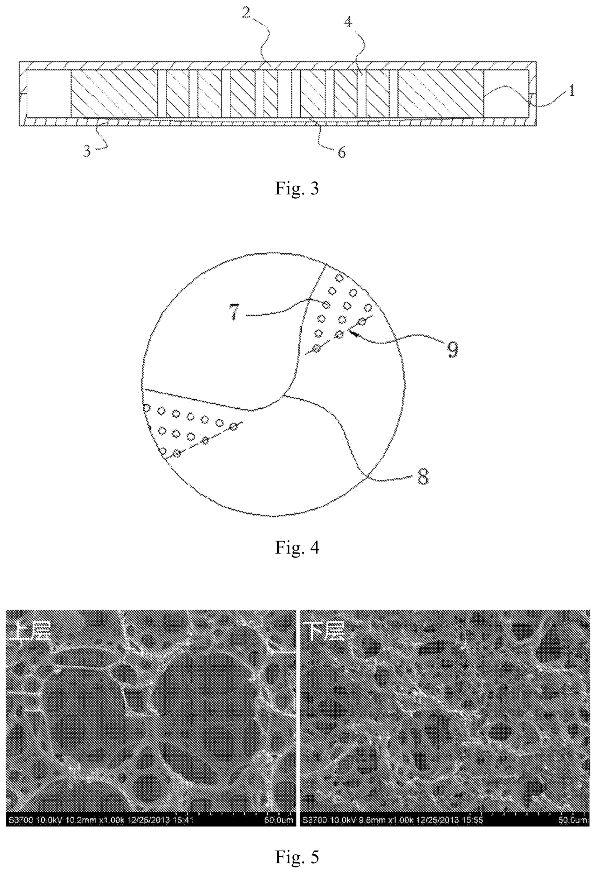

FIG. 5 shows electron microscopy images of the upper permeable layer and the lower permeable layer in Example 1.

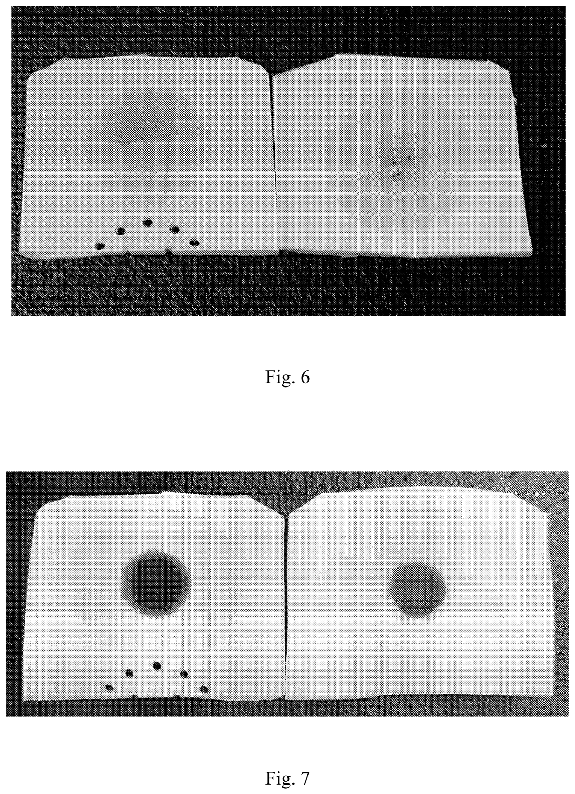

FIG. 6 is a surface-side result image of the blood absorption test in the Test Example;
   wherein the left side shows the test result of Comparative Example 1, and the right side shows the test result of Example 1.
FIG. 7 is a back-side result image of the blood absorption test in the Test Example;
   wherein the left side shows the test result of Comparative Example 1, and the right side shows the test result of Example 1.

### DETAILED DESCRIPTION OF EMBODIMENTS

In order to more clearly illustrate the technical solution of the present invention, the following examples are listed. Unless otherwise specified, the raw materials, reactions, and posttreatment means described in the examples are all common raw materials on the market and technical means well known to those skilled in the art.

The cross-linking agent in the examples of the present invention is ethylene glycol dimethacrylate.

The initiator in the examples of the present invention is ammonium persulfate.

The photoinitiator in the examples of the present invention is 1-hydroxycyclohexyl phenyl ketone (photoinitiator 184).

The emulsifier in the examples of the present invention is diglycerol mono-isostearate and dioctadecyl dimethyl ammonium bromide in a mass ratio of 1:1.

The term "parts" in the examples of the present invention refers to parts by mass.

### Example 1

An absorbent article comprises an absorbent core (1), the absorbent core (1) comprising:
an upper permeable layer and a lower permeable layer, wherein the upper permeable layer and the lower permeable layer are each an integrated polymeric microporous foam structure, a plurality of first permeable pores are provided in the upper permeable layer, a plurality of second permeable pores are provided in the lower permeable layer, and the first permeable pores has a pore size greater than that of the second permeable pores;
the absorbent core (1) is provided with a hydrophilic surface layer (2) on an upper surface thereof and a water-repellent bottom layer (3) on a lower surface thereof, and a non-bonded region is provided between the absorbent core (1) and the water-repellent bottom layer (3), thereby forming a liquid storage cavity (6);
adhesive portions are provided on the side of the water-repellent bottom layer (3) away from the absorbent core (1), and are located on two sides of the liquid storage cavity (6);
the edges of the hydrophilic surface layer (2) and the water-repellent bottom layer (3) are connected by a peripheral sealing embossing layer (5), and the hydrophilic surface layer (2) and the water-repellent bottom layer (3) are bonded to each other by an adhesive layer to enclose the absorbent core (1) between the hydrophilic surface layer (2) and the water-repellent bottom layer (3); a vertical pore region is provided on the absorbent core (1), and an adhesive-free region is provided between the water-repellent bottom layer (3) and the vertical pore region, so that the vertical pore region and the water-repellent bottom layer (3) are not bonded to each other, thereby forming the liquid storage cavity (6);
a plurality of through-holes (10) penetrating the absorbent core (1) are provided in the vertical pore region, a flow guiding region located on the absorbent core (1) is provided on one side of the vertical pore region, a plurality of arc-shaped flow guiding grooves (11) arranged along the length direction of the absorbent core (1) are provided in the flow guiding region, and the lengths of the plurality of arc-shaped flow guiding grooves (11) present a gradual decrease toward the vertical pore region;
the hydrophilic surface layer (2) and the water-repellent bottom layer (3) are both provided with wing portions (4), the adhesive portions comprise first adhesive strips (12) and second adhesive strips (13) provided on the water-repellent bottom layer (3), and the first adhesive strips (12) are located on the wing portions (4), and the second adhesive strips (13) are symmetrically provided on two sides of the liquid storage cavity (6);
the connection locations between the wing portions (4) and the hydrophilic surface layer (2) and between the wing portions (4) and the water-repellent bottom layer (3) are each provided with an arc-shaped corner (8), and the edge of each arc-shaped corner (8) is provided with an embossed separation region, so that the hydrophilic surface layer (2) and the water-repellent bottom layer (3) are both completely separated from the peripheral sealing embossing layer (5) at the edges of the wing portions (4);

The peripheral sealing embossing layer (5) is provided with at least two layers, each layer of the peripheral sealing embossing layer (5) comprises a plurality of uniformly arranged embossing holes (7), the embossing holes (7) of two adjacent layers of the peripheral sealing embossing layer (5) are aligned along common straight lines, and the number of embossing holes (7) in the at least two layers of the peripheral sealing embossing layer (5) adjacent to the embossed separation region decreases in the direction toward the absorbent core (1), so that an inclined line (9) is formed at an end portion of the at least two layers of the peripheral sealing embossing layer (5) adjacent to the embossed separation region;
wherein
the absorbent core (1) is obtained by reacting an acrylate monomer, a modified acrylate, a cross-linking agent, a photoinitiator, an emulsifier, and an initiator.

A method for preparing the modified acrylate comprises the steps of:
S1. adding isosorbide, glycidyl ether (isosorbide:glycidyl ether = 1:1, n/n), and a catalytic amount of tetrabutylammonium bromide into tetrahydrofuran, heating to carry out a reaction at 65°C for 50 h in a nitrogen atmosphere, cooling, and then adding cyclohexane for settling, washing with deionized water and diethyl ether, and then performing vacuum-drying to obtain an intermediate product;
S2. adding the intermediate product and a polymerization inhibitor (hydroquinone in a mass of 1% based on the mass of the system) into a solvent (1,4-dioxane), heating and stirring at 100°C, and adding dropwise a mixed solution of a catalyst DMA and acrylic acid (epoxy groups in the intermediate product:acrylic acid = 2:1, n/n), then measuring the acid value at an interval of 30 min where no further change in the acid value indicates the completion of the reaction, cooling the mixture, and then settling and washing with deionized water, and performing vacuum-drying to obtain the modified acrylate.

A method for preparing the absorbent core (1) comprises the steps of:
L1. mixing 60 parts of isooctyl acrylate, 15 parts of the modified acrylate, 20 parts of the cross-linking agent, 1 part of the photoinitiator, and 5 parts of the emulsifier to obtain an oil phase 1, and then adding a water phase A containing 2% by mass of calcium chloride and a water phase B containing 7% by mass of the initiator, the volume ratio of the oil phase 1, the water phase A, and the water phase B being 1:25:1, so as to obtain a high internal phase emulsion 1;
   specifically, in a continuous emulsification apparatus, the oil phase material was stored in a oil phase material storage tank, the inorganic salt water phase material was stored in a water phase material storage tank, and the initiator water phase material was stored in a initiator storage tank, and during stable operation of the emulsification apparatus, the emulsion part undergoing primary mixing and emulsification in a first static mixer module was delivered back to a first emulsification kettle under the control of a first reflux constant-flow pump, and the emulsion part undergoing secondary mixing and emulsification in a second static mixer module was delivered back to a second emulsification kettle under the control of a second reflux constant-flow pump; the flow rate of the reflux was precisely controlled by the first reflux constant-flow pump, and at the same time, the oil phase in the oil phase material storage tank and the water phase in the water phase material storage tank were continuously delivered to the first emulsification kettle by an oil phase feed constant-flow pump and a water phase feed constant-flow pump, respectively, and were premixed with the emulsion being mixed and emulsified in the first emulsification kettle to obtain a premixed material, which was then delivered to the first static mixer module for mixing and emulsification to obtain a premixed emulsion 1 with a water-oil ratio of 14:1;
   the premixed emulsion 1 was partly delivered to the first emulsification kettle under the control of the first reflux constant-flow pump and partly delivered to the second emulsification kettle under the control of a premixed emulsion 1 feed constant-flow pump, and the premixed emulsion 1 in the premixed emulsion 1 feed constant-flow pump was premixed with the water phase continuously delivered to the second emulsification kettle by the water phase feed constant-flow pump, so as to obtain a premixed material, which was then delivered to the second static mixer module for mixing and emulsification to obtain a premixed emulsion 2; the premixed emulsion 2 was partly delivered to the second emulsification kettle under the control of the second reflux constant-flow pump and partly delivered to a third static mixer module under the control of a constant-flow pump, and was finally mixed and emulsified with the initiator water phase, which was continuously delivered under the control of an initiator feed constant-flow pump, so as to obtain a high internal phase emulsion 1, which was then continuously delivered to the next process via an emulsification product outlet;
L2. mixing 45 parts of isooctyl acrylate, 5 parts of the modified acrylate, 10 parts of the cross-linking agent, 1 part of the photoinitiator, and 2 parts of the emulsifier to obtain an oil phase 2, and then adding a water phase C containing 1% by mass of calcium chloride and a water phase D containing 8% by mass of the initiator, the volume ratio of the oil phase 2, the water phase C, and the water phase D being 1:32:1, so as to obtain a high internal phase emulsion 2;
   the treatment process of the high internal phase emulsion 2 in the continuous emulsification apparatus was the same as that in L1;
L3. coating the high internal phase emulsion 1 onto the lower surface of a non-woven fabric at a thickness of 1.5 mm; coating the high internal phase emulsion 2 onto the upper surface of the non-woven fabric at a thickness of 0.5 mm; and then using light irradiation with a wavelength of 450 nm and an intensity of 50 mW/cm² for 120 s, followed by curing in a steam tunnel oven for 5 min, and then performing dehydration, drying and slitting to obtain the absorbent core.

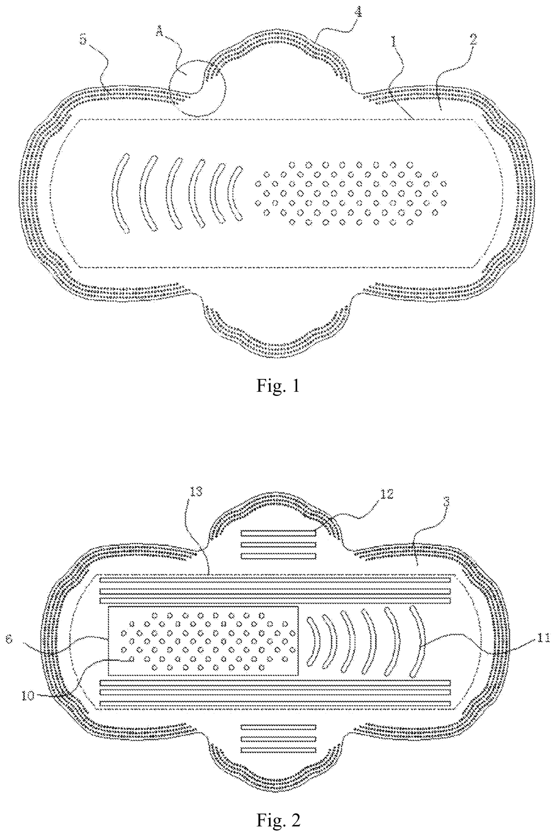

FIG. 1 is a schematic front structural view of an absorbent article of the present invention.

FIG. 2 is a schematic rear structural view of the absorbent article of the present invention.

FIG. 3 is a schematic cross-sectional structural view of the absorbent article of the present invention.

FIG. 4 is an enlarged structural schematic diagram of position A in FIG. 1.

Reference numerals: 1-absorbent core, 2-hydrophilic surface layer, 3-water-repellent bottom layer, 4-wing portion, 5-peripheral sealing embossing layer, 6-liquid storage cavity, 7-embossing hole, 8-arc-shaped corner, 9-inclined line, 10-through-hole, 11-arc-shaped flow guiding groove, 12-first adhesive strip, 13-second adhesive strip.

FIG. 5 shows electron microscopy images of the upper permeable layer and the lower permeable layer in Example 1, and it can be seen that the first permeable pores have a pore size of 60-130 µm, and the second permeable pores have a pore size of 5-40 µm.

### Comparative Example 1

An absorbent article, wherein the difference between this comparative example and Example 1 is that: in steps L1 and L2, the modified acrylate was replaced with an equal mass of acrylate, while all other materials and preparation methods therefor were the same as those of Example 1.

### Comparative Example 2

An absorbent article, wherein the difference between this comparative example and Example 1 is that: in steps L1 and L2, no emulsifier was added, while all other materials and preparation methods therefor were the same as those of Example 1.

### Test example

Performance comparison tests were performed on Example 1 and Comparative Examples 1-2.

Test method: water absorption and blood absorption tests were performed according to GB/T14207-2008.

The test results are shown in Table 1.

**Table 1 Absorption performance test results**

| Item | Example 1 | Comparative Example 1 | Comparative Example 2 |
|---|---|---|---|
| Artificial menstrual blood penetration time (s) | 6.31 | 9.84 | 10.72 |
| Artificial menstrual blood rewet amount (s) | 0.74 | 1.83 | 2.98 |
| Water absorption amount (g) | 51.08 | 45.29 | 36.65 |
| Water absorption ratio (times) | 12.2 | 11.8 | 9.1 |

FIG. 6 is a surface-side result image of the blood absorption test in the Test Example;
wherein the left side shows the test result of Comparative Example 1, and the right side shows the test result of Example 1.

FIG. 7 is a back-side result image of the blood absorption test in the Test Example;
wherein the left side shows the test result of Comparative Example 1, and the right side shows the test result of Example 1.

From Table 1, it can be concluded that the compounding of the modified acrylate and the acrylic monomer as well as other ingredients in Example 1 of the present invention can produce a good synergistic effect, and the prepared absorbent article has excellent liquid absorption capacity, and the penetration time, rewet amount, water absorption amount, and other data thereof are all significantly better than those of Comparative Examples 1-2. In contrast, in Comparative Example 1, due to the replacement of the modified acrylate, it is difficult for the absorbent core obtained by the reaction to form a more three-dimensional and dense microporous structure, resulting in markedly reduced absorption performance of the core and failure to generate ideal capillary action; in Comparative Example 2, the deletion of the emulsifier prevents the formation of a suitable emulsion for polymerization, thereby greatly degrading product performance and resulting in poor liquid conductivity and low absorption efficiency. In addition, in the blood absorption test of Comparative Example 1, obvious liquid leakage and seepage occur, while the absorbent samples prepared in the Example can retain the liquid in the liquid storage cavity, effectively preventing menstrual blood rewetting.

It is apparent to those skilled in the art that the present invention is not limited to the details of the above exemplary examples, and can be implemented in other specific forms without departing from the spirit or essential characteristics of the present invention. Therefore, the examples should be regarded as exemplary and non-limiting in any respect, and the scope of the present invention is defined by the appended claims rather than the above description. Thus, all changes that fall within the meaning and scope of equivalents of the claims are intended to be included within the present invention.

In addition, it should be understood that although the present specification is described according to the embodiments, not every embodiment includes only one independent technical solution, and such description of the specification is only for clarity. Those skilled in the art should consider the specification as a whole, and the technical solutions in the examples can also be appropriately combined to form other embodiments that would be understood by those skilled in the art.

## Claims

1. An absorbent article comprising:
a hydrophilic surface layer, a water-repellent bottom layer, and an absorbent core disposed there between;
wherein the absorbent core comprises a high internal phase emulsion foam comprising an acrylate monomer, a modified acrylate, a cross-linking agent, a photoinitiator, and an emulsifying agent;
wherein the modified acrylate is obtained by reacting a reaction product of isosorbide and glycidyl ether with acrylic acid;
wherein the absorbent core comprises an upper permeable layer and a lower permeable layer, wherein the upper permeable layer comprises a plurality of first permeable holes having a first pore size and the lower permeable layer comprises a plurality of second permeable holes having a second pore size, wherein the first pore size is greater than the second pore size.

2. The absorbent article of claim 1, wherein the glycidyl ether is selected from one or more of triglycidyl ether and pentaerythritol glycidyl ether.

3. The absorbent article of any one of the preceding claims, wherein the emulsifying agent comprises a main emulsifying agent and an auxiliary emulsifying agent.

4. The absorbent article of claim 3, wherein the main emulsifying agent is selected from mono-or poly-stearates, isostearates and oleates of mono-, di-, 3-to 10-polyglycerols, preferably di-glycerol mono-isostearates or oleates and poly-3-glycerol mono-isostearates or oleates.

5. The absorbent article of claim 4, wherein the auxiliary emulsifier is selected from C2-C4 short carbon and C12-C22 long carbon chain aliphatic betaine or quaternary ammonium salt, preferably propyl dimethyl ammonium bromide and dioctadecyl dimethyl ammonium bromide.

6. The absorbent article of any one of the preceding claims, wherein the first pore size is from 60 to 130 µm.

7. The absorbent article of any one of the preceding claims, wherein the second pore size is from 5 to 40 µm.

8. The absorbent article of any one of the preceding claims, wherein the method of preparing the modified acrylate comprises the steps of:
a. adding isosorbide, glycidyl ether and a catalyst into a solvent, heating the mixture to react in an inert gas atmosphere, cooling the mixture, and then settling, washing and drying the cooled mixture to obtain an intermediate product; and
b. adding the intermediate product and a polymerization inhibitor into a solvent, heating and stirring, adding a mixed solution of a catalyst and acrylic acid, reacting, settling, washing and drying to obtain the modified acrylic ester.

9. The absorbent article of claim 8, wherein in step (a), the temperature of the heating reaction is from 60°C to 70 °C and wherein in step (b), the temperature of heating and stirring is 80-100 °C.

10. The method of any one of the preceding claims, wherein the water phase comprises an initiator; wherein the initiator is selected from one or more of ammonium persulfate, sodium persulfate, potassium persulfate, and azo initiator.

11. The method of any one of the preceding claims, wherein the photoinitiator is one or more of benzophenone, benzil, thioxanthone, benzyl ketal, α -hydroxyalkyl benzophenone, α - aminoalkylbenzophenone, and acylphosphine oxide.

12. A method for preparing an absorbent core comprising the steps of:
a. mixing an acrylate monomer, a modified acrylate, a cross-linking agent, a photoinitiator, and an emulsifying agent to form an oil phase;
b. adding a water phase to the oil phase to form a first high internal phase emulsion;
c. depositing the first high internal phase emulsion on a surface;
d. exposing the first high internal phase emulsion to a light treatment;
e. placing the first high internal phase emulsion in a steam tunnel furnace; dehydrating and drying the high internal phase emulsion to obtain the absorbent core; wherein the modified acrylate is obtained by reacting a reaction product of isosorbide
and glycidyl ether with acrylic acid.

13. The method of claim 12, wherein the glycidyl ether is selected from one or more of triglycidyl ether and pentaerythritol glycidyl ether.

14. The method of claim 12 or 13, wherein the water phase comprises an initiator; wherein the initiator is selected from one or more of ammonium persulfate, sodium persulfate, potassium persulfate, and azo initiator.

15. The method of any one of claims 12 to 14, wherein the oil phase comprises 60 parts by weight of isooctyl acrylate, 15 parts by weight of modified acrylic ester, 20 parts by weight of the cross-linking agent, 1 part by weight of the photoinitiator, and 5 parts by weight of an emulsifier.

16. The method of any one of claims 12 to 15, wherein the method of making the modified acrylate comprises the steps of:
a. adding isosorbide, glycidyl ether and a catalyst into a solvent, heating the mixture to react in an inert gas atmosphere, cooling the mixture, and then settling, washing and drying the cooled mixture to obtain an intermediate product; and
b. adding the intermediate product and a polymerization inhibitor into a solvent, heating and stirring, adding a mixed solution of a catalyst and acrylic acid, reacting, settling, washing and drying to obtain the modified acrylic ester.

17. The method of any one of claims 12 to 16, further comprising mixing a second acrylate monomer, a second modified acrylate, a second cross-linking agent, a second photoinitiator, and a second emulsifying agent to form a second oil phase; and adding a second water phase to the second oil phase to form a second high internal phase emulsion.

18. The method of claim 17, wherein the second high internal phase emulsion is deposited on the first high internal phase emulsion.
